# EUROPEAN PATENT APPLICATION

(11) **EP 0 785 180 A2**
(43) Date of publication of application: **23.07.1997**
(21) Application number: 97300199.3
(22) Date of filing: 14.01.1997
(51) Int. Cl.: C07C 37/16, B01J 23/72

(54) **Process for the alkylation of hydroxyaromatic compounds**

(30) Priority: 16.01.1996 US 586389
(71) Applicant: GENERAL ELECTRIC COMPANY, Schenectady, NY 12345 (US)
(72) Inventor: Warner, Gregory Lee, Schenectady, New York 12309 (US); Caruso, Andrew James, Schenectady, New York 12308 (US)
(74) Representative: Szary, Anne Catherine, Dr.

(57) **Abstract**

The reaction rate in the catalytic alkylation of a hydroxyaromatic compound such as phenol with an alkanol such as methanol, especially with the use of a magnesium compound as catalyst, is increased by treatment of the catalyst with carbon monoxide, typically at a temperature of at least about 350°C.

## Description

### Background of the Invention

This invention relates to the alkylation of hydroxyaromatic compounds, and more particularly to the improvement of the activity of catalysts used therein.

The catalytic alkylation of hydroxyaromatic compounds such as phenol with alkanols such as methanol is a known reaction. It is commercially employed for the preparation of such compounds as 2,6-xylenol, a valuable intermediate in the preparation of polyphenylene ethers. Numerous patents disclose catalysts suitable for use in this alkylation process.

In addition to 2,6-alkylation, these catalysts may cause alkylation at other positions, notably at the 4-position to form such compounds as mesitol (2,4,6-trimethylphenol). 4-Alkylated compounds are generally not desirable, however, by reason of the impossibility or at least difficulty of converting them to polyphenylene ethers. Therefore, there have been many developments related to the selectivity of the catalysts. Particularly useful in this respect are the mixed magnesium-copper catalysts disclosed, for example, in US Patents 4,554,266 and 4,554,267, which have good selectivity for 2,6-alkylation.

Catalyst treatment methods which improve the yields of alkylated products without adversely affecting their selectivity would be of great interest. Accordingly, there are continuing studies intended to develop such methods.

### Summary of the Invention

The present invention is based on the discovery that treatment of the aforementioned alkylation catalysts with carbon monoxide significantly improves their efficiency, as demonstrated by improved product yields. Further, it can increase the useful life of the catalyst by periods on the order of 10 hours without an adverse effect on product selectivity. Thus, catalysts affording products containing high proportions of 2,6-xylenol continue to do so even when the initial rate advantage provided upon carbon monoxide treatment is no longer observed.

In one of its aspects, the invention is a method for preparing an alkylated hydroxyaromatic compound by the reaction of a hydroxyaromatic compound with a primary or secondary alkanol in the presence of a metal-containing alkylation catalyst wherein the metal is a non-carbonyl-forming metal, which comprises the step of contacting said catalyst with carbon monoxide for a time and at a temperature effective to saturate said catalyst with carbon monoxide.

Another aspect is a method for maximizing rate enhancement of such a metal-containing catalyst for the alkylation of a hydroxyaromatic compound with an alkanol which comprises the same step.

Still another aspect is a composition comprising such a metal-containing alkylation catalyst which has been contacted with carbon monoxide as described hereinabove.

### Brief Description of the Drawings

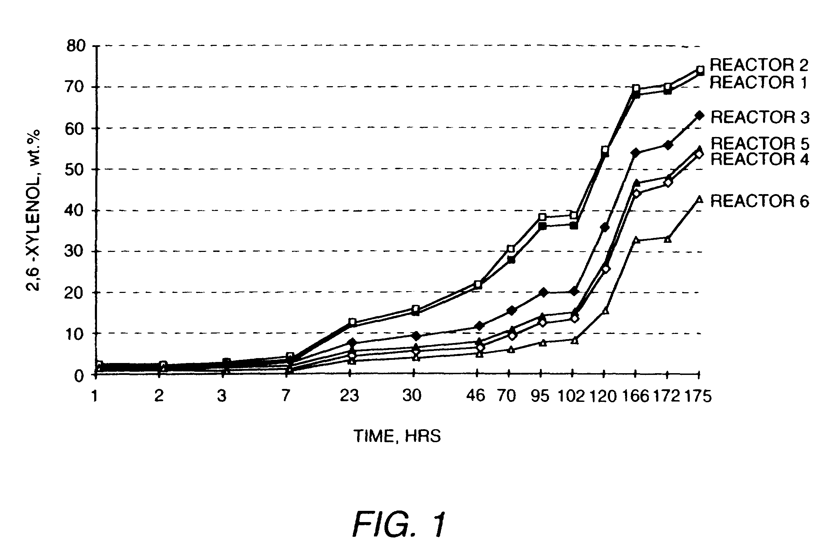

The drawings (FIGURES 1-2) are graphs showing the results of the procedures of the examples herein; namely, the percentages of the desired 2,6-xylenol in the product streams from the alkylation of phenol with methanol.

### Detailed Description; Preferred Embodiments

The hydroxyaromatic compounds which may be alkylated according to the present invention include all of such compounds which have a free ortho-position. Illustrative hydroxyaromatic compounds are phenol, 1-naphthol, 2-naphthol, o-cresol, m-cresol, p-cresol, catechol, resorcinol and hydroquinone. Monohydroxyaromatic compounds are preferred, with phenol and o-cresol being most preferred. o-Cresol is obtained in substantial yield as a by-product in the alkylation of phenol and may be recycled for further alkylation to 2,6-xylenol.

The alkanol used for alkylation may be primary or secondary and is usually primary. It is most often a C₁₋₇ alkanol, with methanol being preferred.

Alkylation catalysts are disclosed in many patents. They include various compounds, chiefly oxides, of such metals as magnesium, calcium, zinc, tellurium and copper. Magnesium oxides are often preferred, with copper-modified magnesium oxides being most preferred. Copper contents up to about 0.1% by weight and especially in the range of about 0.02-0.04% by weight are typical. The catalyst may also contain at least one filler such as graphite or polyphenylene ether.

Although such catalyst metals as chromium, manganese, nickel and iron are disclosed as being present in some alkylation catalysts, the presence of such carbonyl-forming metals in the catalyst employed in this invention is not contemplated. This is because the reaction of said metals with carbon monoxide to form metal carbonyls tends to foul the catalyst. In addition, many of the metal carbonyls are highly volatile and/or toxic.

In the preparation of the catalyst, a final step is usually calcination at a temperature typically in the range of about 350-550°C, most often in an inert or reducing atmosphere such as hydrogen or nitrogen. The invention contemplates treatment of the catalyst with carbon monoxide after calcination.

Carbon monoxide treatment is usually a preliminary step prior to alkylation. However, it is also within the scope of the invention to simultaneously contact the catalyst with carbon monoxide and alkanol. Said treatment may be at atmospheric or superatmospheric pressure, typically at pressures in the range of about 1-5 atm.

The time temperature of treatment with carbon monoxide should be effective to saturate the catalyst therewith. Saturation is indicated by maximization of the alkylation rate, and can be detected by simple experimentation. In general, saturation is more quickly completed at lower temperatures. However, the practicalities of the alkylation process may dictate employment of higher temperatures and this is in no way detrimental to the reaction.

Treatment temperatures are typically in the range from about 350°C to just below the sintering temperature of the catalyst mass, especially about 350-450°C. Most often, the carbon monoxide is passed over the catalyst mass for a period of time effective to achieve thorough, intimate contact with the entire mass, with times of about 3-30 hours being typical and about 10-15 hours being preferred.

The alkylation reaction, usually following but sometimes concurrent with carbon monoxide treatment, is conducted under conventional conditions. These generally include pressures in the range of about 1-10 atm and temperatures in the range of about 400-500°C, preferably about 420-460°C. Typically, a reactant stream of alkanol and hydroxyaromatic compound in a molar ratio of about 3-6:1 (respectively) is passed over the catalyst mass in the gas phase. The reactant stream may also contain water as a diluent and flow control agent. The product stream is most often isolated by liquefaction upon cooling and is distilled to separate the constituents thereof.

The invention is illustrated by the following examples.

### Example 1

Pellets of magnesium oxide/copper catalyst and graphite and polyphenylene ether as fillers (88 g), prepared by a method similar to that of Example 4 of US Patent 4,554,267, were charged to six identical stainless steel reactors (Reactors 1-6) with inner diameters of 2.1 cm to a catalyst bed depth of 33 cm. The exit tube from each reactor was fitted with a control valve and a pressure transducer capable of maintaining a modest back pressure. Each exit tube was equipped with a sampling valve and condenser.

Reactors 1-3 were heated to 380°C and reactors 4-6 to 420°C in a salt bath and were allowed to equilibrate for one hour. A stream of nitrogen (135 ml/min) was passed through each reactor for 24 hours, after which the temperature was raised to 440°C to complete calcination of the catalyst. The following procedures were then performed.

Reactors 1 and 5: The reactors were purged with carbon monoxide (flow rate 100 ml/min) at 440°C and atmospheric pressure for 24 hours. A reactant stream of methanol and phenol in a 4:1 molar ratio and 20% (by weight) water was then introduced at a LHSV (liquid hourly space velocity) of 2.2 liters feed per liter catalyst per hour and a back pressure of 2.7 atm abs. The exit streams were analyzed at intervals by gas chromatography.

Reactors 2 and 4: The reactors were purged with carbon monoxide at 440°C, a flow rate of 100 ml/min and a back pressure of 3.72 atm abs for 24 hours, after which the reactant stream was introduced as described for Reactors 1 and 5.

Reactors 3 and 6 (controls): The reactant stream was introduced as described for Reactors 1 and 5 without carbon monoxide treatment.

The results of these runs are presented graphically in FIGURE 1. It is apparent that the normalized percentages of 2,6-xylenol in the product mixture were approximately the same irrespective of the pressure of carbon monoxide treatment, and were substantially greater when operating according to the invention than in the controls.

### Example 2

Four reactors (Reactors 7-10) were charged with about 90 g each of catalyst, heated in a salt bath at 390°C and purged with nitrogen for 12 hours, as described in Example 1. Reactors 8-10 were then allowed to stand at room temperature for 12, 18 and 21 hours, respectively.

Reactors 7-10 were pressurized briefly with carbon monoxide at room temperature and 1.14 atm abs and vented, then pressurized with carbon monoxide at 390°C and 0.34 atm, with high temperature carbon monoxide treatment being timed to end simultaneously for all four reactors. Thus, the carbon monoxide treatment times for Reactors 7-10 were 24, 12, 6 and 3 hours respectively. The salt bath temperatures were then increased to 450°C and the reactant stream described in Example 1 was passed through each reactor at 4.0 ml/min and a back pressure of 2.7 atm abs.

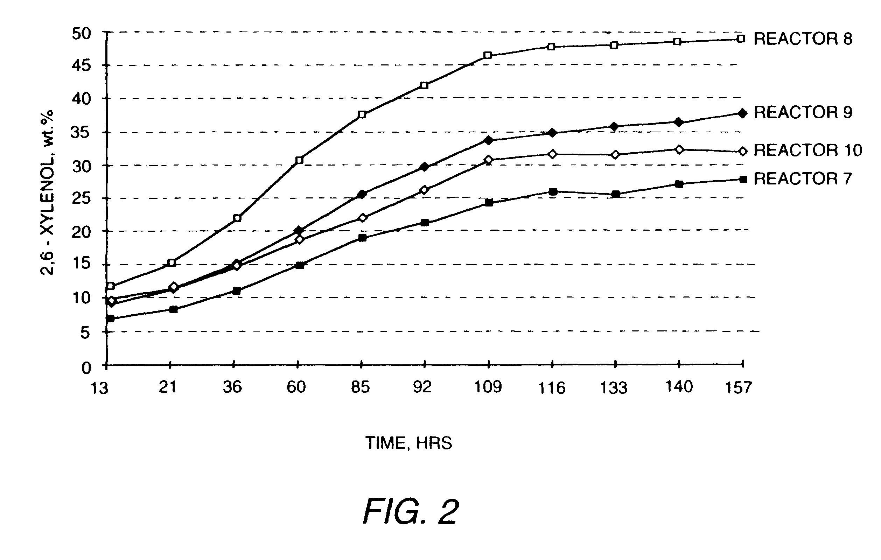

The results of periodic analysis of the product streams are presented graphically in FIGURE 2, corrected for differences in catalyst weights in the four reactors. It is apparent that the carbon monoxide treatment time of 12 hours (Reactor 8) afforded the highest yield of 2,6-xylenol.

## Claims

1. A method for preparing an alkylated hydroxyaromatic compound by the reaction of a hydroxyaromatic compound with a primary or secondary alkanol in the presence of a metal-containing alkylation catalyst wherein the metal is a non-carbonyl-forming metal, which comprises the step of contacting said catalyst with carbon monoxide for a time and at a temperature effective to saturate said catalyst with carbon monoxide.

2. A method according to claim 1 wherein the catalyst is a magnesium compound.

3. A method according to claim 2 wherein the catalyst is a copper-modified magnesium oxide.

4. A method according to claim 2 wherein said catalyst is contacted with carbon monoxide prior to alkylation.

5. A method according to claim 2 wherein the contact temperature is in the range of about 350-450°C.

6. A method according to claim 5 wherein the contact is at atmospheric or superatmospheric pressure.

7. A method according to claim 2 wherein the alkanol is methanol.

8. A method according to claim 7 wherein the hydroxyaromatic compound is phenol or o-cresol.

9. A method for maximizing rate enhancement of a metal-containing alkylation catalyst wherein the metal is a non-carbonyl-forming metal for the alkylation of a hydroxyaromatic compound with an alkanol which comprises contacting said catalyst with carbon monoxide for a time and at a temperature effective to saturate said catalyst with carbon monoxide.

10. A composition comprising a metal-containing alkylation catalyst wherein the metal is a non-carbonyl-forming metal, which catalyst has been contacted with carbon monoxide at a temperature of at least about 350°C.
